# EUROPÄISCHE PATENTSCHRIFT

(11) **EP 1 752 170 B1**
(45) Veröffentlichungstag und Bekanntmachung des Hinweises auf die Patenterteilung: **13.06.2012**
(21) Anmeldenummer: 06023095.0
(22) Anmeldetag: 28.07.2001
(51) Int. Cl.: A61L 31/04

(54) **Medizintechnisches Produkt, Verfahren zu seiner Herstellung und Bereitstellung für die Chirurgie**
Medical technical product, method of production and provision in surgery
Produit médico-technique, procédé pour sa production et son utilisation

(30) Priorität: 02.08.2000 DE 10037601; 06.04.2001 DE 10117099
(43) Veröffentlichungstag der Anmeldung: 14.02.2007
(62) Teilanmeldung aus: 01978270.5
(73) Patentinhaber: Aesculap AG, 78532 Tuttlingen (DE)
(72) Erfinder: Frierich, Volker, 78579 Neuhausen (DE); Odermatt, Erich K., 8200 Schaffhausen (CH); Weis, Christine, 78532 Tuttlingen (DE)
(74) Vertreter: Patentanwälte Ruff, Wilhelm, Beier, Dauster & Partner

(56) Entgegenhaltungen:
- WO-A-98/12243
- WO-A-98/16265
- US-A- 5 393 528

## Beschreibung

Die vorliegende Erfindung betrifft ein medizintechnisches Produkt zur Adhäsionsprophylaxe für die postoperative Verhinderung von Verwachsungen im Körper, ein Verfahren zu seiner Herstellung und seine Bereitstellung für die Chirurgie.

Bei jedem chirurgischen Eingriff in die Leibeshöhle eines Patienten besteht die Gefahr von unerwünschten Narbenbildungen. Die Operation stellt eine Traumatisierung des Peritoneums dar, die zu einer entzündlichen Reaktion mit Exsudatiön von Fibrin führt. Daraus entstehende fibrinöse Verklebungen können bei ausreichender fibrinolytischer Aktivität innerhalb der Bauchhöhle aufgelöst werden, so dass keine andauernden Beschwerden beim Patienten auftreten. Reicht aber die enzymatische Reaktion bei einem Patienten nicht aus, um die Fibrinablagerungen zu lysieren, entstehen feste permanente Adhäsionen. Solche Verwachsungen können beim betroffenen Patienten zu Schmerzen, verzögerter Wundheilung oder Funktionsstörungen an Bauchorganen führen.

Als Beispiele sind allgemein chirurgische Eingriffe und auch Laparoskopien im Bereich der Gynäkologie zu nennen, wo entstandene Verwachsungen häufig Ursache von Unterleibsschmerzen und Infertilität sind.

In der Herzchirurgie werden in den letzten Jahren zunehmend Reoperationen, Ersatz von Kunstklappen oder Anlage aortocoronarer Bypasse durchgeführt. Hierbei stellen durch einen Primäreingriff hervorgerufene pericardiale Adhäsionen, wegen Blutungskomplikationen oder Verletzungen aortocoronarer Bypässe ein erhebliches Risiko für den Patienten mit hoher Letalitätsrate dar.

In der Nephrologie können peritoneale Adhäsionen bei der Durchführung der kontinuierlich ambulanten Peritonealdialyse entstehen. Bei Verwachsungen im Darmbereich werden häufig Passagestörungen beobachtet.

Diese Komplikationen führen neben dem subjektiven Krankheitsgefühl des Patienten, zu verlängerter medizinischer Behandlung, chronischen Schmerzen und erhöhten Kosten.

Seit langem versucht die Medizin, durch Verbesserung operationstechnischer Massnahmen und der chirurgischen Technik, Verwachsungen und die damit verbundenen Komplikationen zu verhindern. Diese reichen jedoch nicht aus, so dass wirksame additive Massnahmen zur Prophylaxe postoperativer Adhäsionen notwendig sind.

Es wurden zur Prophylaxe peritonealer Adhäsionen eine Vielzahl von Substanzen vorgeschlagen, wie beispielsweise Glaskörperflüssigkeit von Kalbsaugen oder Olivenöl. Mit Antikoagulantien und Fibrinolytika konnte zwar eine Reduzierung der Verwachsungen erreicht werden, das Risiko postoperativer Blutungen hat jedoch eine Verbreitung in den Kliniken verhindert. Die lokale Anwendung von Antibiotika hat überwiegend zu einer Verstärkung der Adhäsionsbildungen geführt. Die meisten bekannten Substanzen zur Adhäsionsprophylaxe werden mit dem Ziel eingesetzt, die Läsionen des Peritoneums während der Abheilung von den benachbarten Geweben zu trennen.

Die Erfindung stellt sich die Aufgabe, ein medizintechnisches Produkt zum Einsatz bei chirurgischen Eingriffen, insbesondere zur Vermeidung von unerwünschten Adhäsionen zur Verfügung zu stellen, das die Schwierigkeiten von medizintechnischen Produkten aus dem Stand der Technik überwindet, einfach und kostengünstig herzustellen ist und in der Praxis bei üblichen chirurgischen Methoden leicht zu handhaben ist.

Die Aufgabe wird gelöst durch ein medizintechnisches Produkt zur Adhäsionsprophylaxe für die postoperative Verhinderung von Verwachsungen im Körper umfassend einen physikalisch vernetzten PVA (Polyvinylalkohol) mit einem Molekulargewicht von 15000 bis 400000 g/mol. Mit Vorteil kann das erfindungsgemässe medizintechnische PVA-Produkt als Folie, Membran, Lösung, Schaum, Gel oder als Spray oder Pulver vorgesehen sein.

PVA ist wegen seiner ausgezeichneten Bioverträglichkeit (Biokompatibilität) für eine Anwendung im Körper eines Patienten besonders geeignet. Beispielsweise wird PVA in vivo nicht modifiziert, es führt zu keinen entzündlichen Reaktionen und es wird nur in geringem Umfang in Körperorganen akkumuliert. Prinzipiell ist PVA ein biologisch abbaubares synthetisches Polymer mit einem Kohlenstoff-Kohlenstoff-Rückgrat. Es ist ein enzymatischer Abbaumechanismus bekannt, der mehrere Tage dauert. Die Hydroxylgruppe wird dabei zu einer Ketogruppe oxidiert und unter Spaltung der Kohlenstoff-Kohlenstoff-Bindung in Methylketone und Carboxylsäuren hydrolysiert.

Die Ausscheidung des PVA ohne Degradation erfolgt grösstenteils über die Niere, wobei die Ausscheidungsgeschwindigkeit vom Molekulargewicht abhängig ist. PVA mit einem Molekulargewicht von weniger als 15000 g/mol wird innerhalb weniger Stunden aus dem Körper ausgeschieden, was für die vorgesehene Anwendung in der Adhäsionsprophylaxe zu schnell erfolgt.

Mit besonderem Vorteil kann der erfindungsgemäss eingesetzte PVA ein Molekulargewicht von 20000 bis 400000 g/mol aufweisen. In einer Ausführungsform kann der PVA aus einem Gemisch von niedermolekularen und hochmolekularen Komponenten gebildet sein, wobei insbesondere die hochmolekulare Komponente PVA ist.

Polyvinylalkohol ist wegen seiner Hydroxylgruppen wasserlöslich. Durch eine Modifizierung können die Löslichkeit und andere chemische und physikalische Eigenschaften des Polymers verändert werden. Die Modifizierung von PVA kann durch chemische Modifizierung, durch physikalische Modifizierung oder eine Kombination von Modifizierungen vorgenommen sein. Als Beispiele für chemische Modifizierung sind Copolymerisation, Pfropfung oder chemische Vernetzung zu nennen. Als Beispiel für physikalische Modifizierung sind physikalische Vernetzung, Ausbildung orientierter Molekularstrukturen, Hydrogele und Kristallitbildung zu nennen.

Eine chemische Vernetzung des rohen PVA kann allgemein über die alkoholischen Gruppen in einer Additionsreaktion mit Diisocyanat oder in einer Kondensationsreaktion mit einer mehrfunktionellen Säure durchgeführt werden. Eine reversible Unlöslichkeit durch Vernetzung ist zur biologischen Ausscheidung des PVA bevorzugt. Von besonderer Bedeutung für die Bereitstellung des erfindungsgemässen Produkts für die Medizin ist, dass unter den physiologischen Bedingungen im Körper des Patienten keine toxischen oder gesundheitsschädlichen Substanzen entstehen. Aus diesem Grund ist eine hydrolysierbare Vernetzung, insbesondere mittels mehrwertiger Carbonsäuren, beispielsweise in Form von Anhydriden, erfindungsgemäss bevorzugt. Es kann auch eine enzymatisch spaltbare Vernetzung verwendet werden. Die Vernetzung kann mit einem metabolisierbaren Diisocyanat vorgenommen sein, das eine spaltbare Bindung trägt, wie beispielsweise eine Esterbindung. So können keine Urethanbrücken entstehen, die nicht oder erst nach langer Zeit im Körper abgebaut werden.

Erfindungsgemäss bevorzugt können als Vernetzer mehrwertige Dicarbonsäuren und/oder ihre Derivate vorgesehen sein. Die Veresterung der alkoholischen Gruppen im PVA mit Dicarbonsäuren zeichnet sich als reversible Vernetzungsreaktion aus. Als Vernetzungsmittel können insbesondere Anhydride von Carbonsäuren verwendet sein. Als Beispiele solcher Vernetzungsmittel sind Bernsteinsäureanhydrid oder Oxalsäureanhydrid zu nennen. Bernsteinsäureanhydrid ist reaktiver als die Bernsteinsäure, da Ringöffnungsenergie frei wird.

Auch andere Vernetzer mit mindestens zwei funktionellen Gruppen sind möglich. Ferner können als Vernetzungsmittel Verbindungen mit Doppelbindungen eingesetzt werden. Als Beispiele hierfür sind Imide und Acrylate zu nennen.

Wenn die Vernetzungsreaktion in Lösung durchgeführt wird, ist ein kurzkettigeres Vernetzungsagens vorteilhafter, da die Wahrscheinlichkeit einer intramolekularen Reaktion mit nur einer Polymerkette gering ist. Auf diese Weise sind Oxalsäure und ihre Derivate wegen der kürzeren Molekülkette erfindungsgemäss bevorzugt.

Gemäß der Erfindung ist PVA mit einem Molekulargewicht von 15000 bis 400000 g/mol physikalisch vernetzt. Mit Vorteil kann die physikalische Vernetzung durch Kristallitbildung vorgenommen sein. Bei einer solchen physikalischen Vernetzung ist ein dreidimensionales Netzwerk von PVA-Molekülen ausgebildet, das durch Kristallite als physikalische Vernetzungspunkte zusammengehalten wird.

Zur Ausbildung einer physikalischen Vernetzung kann eine wässrige Lösung von PVA für 6 bis 48 Stunden bei -20 °C eingefroren und dann bei 25 °C über 2 bis 6 Stunden aufgetaut werden, so dass sich die gewünschte Vernetzung ausbildet. Gemäss der Erfindung kann die Darstellung von PVA-Hydrogelen über einen Gefrier-/Tauzyklus vorgenommen werden, der mit Vorteil insbesondere mehrmals wiederholt werden kann. Bei der physikalischen Vernetzung durch Gefrier-/Tauzyklen können bei unterschiedlichen Temperaturen verschiedene Phasen zugeordnet sein. Insbesondere können durch Gefrier-/Tauzyklen Nanopartikel hergestellt werden.

Die PVA-Ketten können auch modifiziert sein, indem nur wenige Hydroxylgruppen des PVA, vorzugsweise über Ester- und/oder Ethergruppen mit zusätzlichen Resten verbunden sind. Für eine solche Modifikation eignen sich insbesondere Fettsäuren und/oder Alkohole mit einer Kettenlänge von C2 bis C16. Es können auch Aminosäuren oder Peptide verknüpft werden. Es reichen 1 bis 10, insbesondere 1 bis 2 Reste pro Molekül PVA aus. Durch die Modifikation kann erreicht werden, dass aus einer PVA-Lösung bei Erwärmung auf Körpertemperatur eine Gelierung erfolgt. Wird beispielsweise eine PVA-Lösung bei Raumtemperatur appliziert, z. B. durch Sprühen, bildet sich bei Erwärmung auf Körpertemperatur (37 °C) sofort ein Film, der die gewünschte Adhäsionsprophylaxe bewirkt.

In einer anderen Ausführungsform kann PVA in Mischung mit einer hochmolekularen Komponente vorliegen, die kein PVA ist. Eine solche hochmolekulare Komponente kann in einer Menge von 0,5 bis 4 Gew.-%, insbesondere 1 bis 2 Gew.-% vorliegen. Die hochmolekulare Komponente kann als Bilayer auf PVA aufgebracht sein. Eine solche Schichtstruktur kann beispielsweise durch Aufsprühen der hochmolekularen Komponente auf eine PVA-Membran ausgebildet sein. Ferner kann die nicht aus PVA gebildete hochmolekulare Komponente zur zusätzlichen Medikamentenaufnahme vorgesehen sein.

Mit Vorteil kann dem erfindungsgemässen PVA als hochmolekulare Komponente ein Zuckerpolymer zugesetzt sein. Insbesondere kann das Zuckerpolymer ausgewählt sein aus der Gruppe bestehend aus Carboxymethylcellulose, Dextran, Hydroxymethylcellulose, Hydroxyethylstärke, Chitosan und/oder Heparin.

Gemäss einer Ausführungsform der vorliegenden Erfindung kann das Produkt zur Adhäsionsprophylaxe in Form einer mindestens einschichtigen Folie vorliegen. PVA-Filme können industriell durch Extrusion hergestellt sein. Insbesondere können PVA-Filme nach einem Giessverfahren hergestellt sein. Besonders dünne und homogene Membranen von etwa 5 bis 7 µm Dicke können nach dem Spin-Casting-Verfahren hergestellt sein.

Erfindungsgemäss kann die Folie aus nur einer Schicht gebildet sein. In einer anderen Ausführungsform der Erfindung kann die Folie aus zwei Schichten, einem sogenannten Bilayer, gebildet sein. Als Beispiele für einen solchen Bilayer sind zwei Schichten auf Basis von PVA oder eine Kombination von PVA und einer weiteren Komponente ausgewählt aus der Gruppe der Kohlenhydrate, Lipide und Proteine sowie deren Derivate zu nennen. Bevorzugt kann ein Bilayer aus PVA und CMC (Carboxymethylcellulose) gebildet sein. In einer weiteren Ausführungsform der Erfindung kann die Folie aus drei Schichten, einem sogenannten Trilayer, gebildet sein. Beispiele hierfür sind sandwichartige Kombinationen von PVA und CMC, etwa mit der Schichtenfolge CMC/PVA/CMC, PVA/CMC/PVA, CMC/PVA/PVA oder CMC/CMC/PVA, ebenso Kombinationen von PVA, CMC und einer weiteren Komponente ausgewählt aus der Gruppe der Kohlenhydrate, Lipide und Proteine sowie deren Derivate.

In sandwichartigen Strukturen können die aus PVA, CMC und gegebenenfalls anderen Komponenten gebildeten Schichten miteinander verbunden sein, beispielsweise durch Verkleben oder Verschweissen. Als Klebemittel sind Wasser, wässrige Lösungen von PVA oder CMC zu nennen. Verschweissen kann durch Anwendung von Wärme, bevorzugt in Form von Punktschweissen, oder Ultraschall erfolgen.

Liegt die mit der PVA-Schicht verbundene zweite Schicht in Form eines offenporigen Materials, beispielsweise in der bevorzugten Form eines insbesondere durch Lyophilisation erhaltenen Vlieses vor, dann braucht die Sandwich- oder Bilayerstruktur nicht besonders am Gewebe des Patienten befestigt zu werden, da die offene Struktur eine Selbsthaftung bewirkt.

Ein schichtförmiges Produkt zur Adhäsionsprophylaxe kann in nicht gepresster Form vorliegen. In einer anderen Ausführungsform kann ein schichtförmiges Produkt zur Adhäsionsprophylaxe in gepresster Form vorliegen. Durch das Pressen kann insbesondere eine oberflächliche Strukturierung, beispielsweise eine Prägung aufgebracht sein.

Erfindungsgemäss kann die Folie mindestens einseitig eine Strukturierung aufweisen. Eine solche Struktur kann beispielsweise durch Giessen auf eine strukturierte Fläche oder durch Prägen ausgebildet sein. Vorzugsweise kann eine Strukturierung in geometrischen Formen vorgesehen sein. Eine solche Oberflächenstruktur kann auf beiden Seiten des schichtförmigen Produkts unterschiedlich gestaltet sein.

Gemäss einer anderen Ausführungsform der vorliegenden Erfindung kann das Produkt zur Adhäsionsprophylaxe in Form eines Schaums oder eines Schaumprecursors vorliegen. In einer besonderen Ausführungsform kann mindestens eine Schicht in Form eines Schaums oder eines Schaumprecursors vorgesehen sein.

Gemäss einer weiteren Ausführungsform der vorliegenden Erfindung kann das Produkt zur Adhäsionsprophylaxe in Form einer Lösung vorliegen. Bevorzugt kann die Lösung gesprayt werden.

Gemäss noch einer anderen Ausführungsform der vorliegenden Erfindung kann das Produkt zur Adhäsionsprophylaxe in Form eines Gels vorliegen. Insbesondere kann es in Form eines Mikrogels vorliegen. Bevorzugt kann es in Form eines formstabilen Hydrogels vorliegen. Mikrogele können mit geeigneten Treibgasen versprüht werden, so dass sie auf diese Weise als Sprühgele für die Adhäsionsprophylaxe verwendet werden können.

Besonders bevorzugt kann der erfindungsgemässe PVA in Form von Mikropartikeln mit Durchmessern in Nanometer-Bereich, sogenannten Nanopartikeln vorliegen. Die Nanopartikel können in verschiedenen Formen als medizintechnisches Produkt vorgesehen sein. Als Beispiele sind die oben genannten Anwendungsformen wie Gel, Folie oder Spray zu nennen. Es ist auch möglich, Mikro-, insbesondere Nanopartikel, auf der Oberfläche einer Folie vorzusehen.

Das erfindungsgemässe medizintechnische Produkt zeichnet sich mit Vorteil dadurch aus, dass es in mit wässrigen Medien gequollener Form bereitgestellt ist. Bei schwach vernetzten, trockenen Polymeren tritt in wässrigen Medien oder geeigneten Lösemitteln Quellung auf, wobei die Quellung an der Oberfläche beginnt und ins Innere fortschreitet. Die Quellungsgeschwindigkeit wird dabei nicht durch die Diffusionskoeffizienten der Quellungsmittel, sondern durch die Diffusionsgeschwindigkeit der Segmente des Polymeren beeinflusst. Je mehr die Quellung fortschreitet, um so stärker machen sich die elastischen Rückstellkräfte der vernetzten Polymerketten bemerkbar. Gele weisen viskoelastische Eigenschaften auf. Die Quellung erfolgt bis zu einem maximalen Wert. Im Gegensatz zu unvernetzten Polymeren, aus denen wässrige Lösungen in variabler Konzentration hergestellt werden können, werden chemisch vernetzte Polymere nicht gelöst.

Das erfindungsgemässe Produkt zur Adhäsionsprophylaxe kann in Form einer im Laminarflow getrockneten Membran durch Quellung eine Flüssigkeitsmenge, wie beispielsweise Wasser, von 20 % des Produktgewichts aufgenommen haben. Durch die Aufnahme von Flüssigkeit bei der Quellung entsteht ein Gel, das reibungshemmende Funktionen ausübt. Das Produkt zur Adhäsionsprophylaxe kann in Form eines Hydrogels zu 80 % aus Wasser bestehen. Für die erfindungsgemässe Anwendung in der Adhäsionsprophylaxe kann es damit die Mesothelfunktion übernehmen.

Für die Verwendung in der Adhäsionsprophylaxe ist es wünschenswert, dass sowohl Membranen als auch Gele nicht steif sind. Die Steifigkeit der Gele ist durch ihren Elastizitätsmodul gegeben, welcher direkt proportional zu der Konzentration an elastischen Netzwerkketten ist. Kovalent vernetzte Gele weisen eine Dimensionalität von d = 3 auf. Der Elastizitätsmodul nimmt mit steigender Konzentration gemäss der Formel E = K·C^{9/4} zu.

Kovalent und physikalisch vernetzte Gele unterscheiden sich in der Konzentrations- und der Polymerisationsgradabhängigkeit der Moduln. Prinzipiell kann man sich physikalische Netzwerke wie einen Teller Spaghetti vorstellen, die sich untereinander verhaken. In beiden Fällen wird der Modul durch die Konzentration an Netzstellen kontrolliert. Bei kovalent verknüpften Netzwerken ist dafür nur der Quellungsgrad und somit die Konzentration des Vernetzers im Gel verantwortlich. Der Polymerisätionsgrad ist unendlich hoch und kann auch durch Scherung nicht verändert werden. Dies spielt eine grosse Rolle bei normalen Körperbewegungen, da der Polymerisationsgrad unverändert bleibt. In kovalenten Netzwerken ist kein Abgleiten der Ketten möglich. Bei physikalischen Netzwerken werden unter Scherung die Netzpunkte aufgelöst und wieder verknüpft. Ein nur physikalisch vernetztes Polymer kann unter bestimmten Bedingungen von einer Oberfläche wieder abgewaschen werden und so in der Chirurgie insbesondere für eine geringe Verweilzeit und geringe Funktionsdauer im Körper geeignet sein. Ein besonderer Vorteil kovalent vernetzter Polymere ist, dass ihre Eigenschaften nach Wunsch eingestellt werden können.

Polyvinylalkohol in Form eines Hydrogels ist ein gummiartiges Material, wobei seine Elastizität über definierte Netzpunkte so eingestellt werden kann, dass sie weichem Körpergewebe oder Muskeln sehr nahe kommt. Gleichzeitig weist PVA eine hohe Zugfestigkeit auf.

Gemäss der Erfindung kann das Molekulargewicht des PVA bzw. der Mischung so gewählt sein, dass er im wesentlichen ohne Degradation der PVA-Moleküle über die Niere ausscheidbar ist. Gegebenenfalls kann PVA nach einer Hydrolyse bzw. nach Aufhebung der Vernetzung, im wesentlichen ohne Degradation der PVA-Moleküle über die Niere ausscheidbar sein.

Erfindungsgemäss bevorzugt kann der Vernetzungsgrad des medizintechnischen PVA-Produkts so eingestellt sein, dass seine Funktionsdauer im Operationsgebiet 5 bis 21 Tage, insbesondere 5 bis 14 Tage beträgt. Zur Adhäsionsprophylaxe ist es wünschenswert, dass das Material mindestens für 5 Tage in der peritonealen Höhle verbleibt. Eine Steuerung der Ausscheidungszeit kann über eine Einstellung von Parametern erfolgen, die die PVA-Ausscheidung beeinflussen. Als Einflussfaktoren sind beispielsweise chemische Vernetzung, physikalische Vernetzung des PVA-Materials, Schichtdicke, Mischungszusammensetzung sowie Zusatz von Additiven wie Zuckerpolymeren zu nennen.

Mit Vorteil kann die makroskopische Auflösung des erfindungsgemässen medizintechnischen Produkts unter physiologischen Bedingungen 7 bis 60 Tage betragen. Die Verweilzeit des PVA im Körper hängt auch ab vom hydrodynamischen Radius der Polymermoleküle. Erfindungsgemässer PVA kann mit Vorteil einen hydrodynamischen Radius von 5 nm bis 15 nm, insbesondere von 5 nm aufweisen.

Mit besonderem Vorteil kann der vernetzte PVA ein in physiologischer Umgebung vorteilhaftes Benetzungsverhalten aufweisen. Das erfindungsgemässe medizintechnische Produkt zur Adhäsionsprophylaxe kann sich besonders dadurch auszeichnen, dass der vernetzte PVA eine Struktur aufweist, die in physiologischem Milieu einen Stoffaustausch von kleinen Molekülen erlaubt, aber eine Anlagerung von physiologischen Substanzen, wie insbesondere Blut und Zellen im wesentlichen verhindert.

Zu diesem Zweck können Struktureigenschaften des medizintechnischen Produkts wie die Oberflächenstruktur, Porenstruktur und die Netzwerkdichte einer PVA-Membran so angepasst sein, dass sie für kleine Moleküle wie beispielsweise Wasser, Glucose oder Nährstoffe durchlässig ist, andererseits aber die Anlagerung von grossen Partikeln, wie beispielsweise Blut, von Körperzellen, Makrophagen oder Mikroorganismen, wie beispielsweise pathogenen Bakterien vermieden werden kann.

PVA-Lösungen und Blends können ein LCST-Verhalten (lower critical solution temperature) aufweisen. Unterhalb dieser kritischen Temperatur liegt das PVA gelöst vor. Oberhalb dieser Temperatur fällt das PVA-Polymer aus. Mittels Modifikationen kann das Phasenverhalten gesteuert werden. Als Beispiele solcher Modifikationen können Anbinden von kurzen Resten mit einer Kettenlänge von C2 bis C16, z. B. Dodecylreste, oder kurzkettigen Fettsäuren, Kohlenhydraten, Aminosäuren, Peptiden oder Blenden, d. h. Mischen mit anderen Polymeren genannt werden. Auf diese Weise kann eine PVA-Lösung bei Raumtemperatur nach Applikation beim Patienten, z. B. Sprühen, auf Körpergewebe bei Körpertemperatur 37 °C sofort einen Film bilden.

Die vorliegende Erfindung betrifft ferner ein Verfahren zur Herstellung eines medizintechnischen Produkts zur Adhäsionsprophylaxe, das dadurch gekennzeichnet ist, dass es aus einem physikalisch vernetzten PVA mit einem Molekulargewicht von 15000 bis 400000 g/mol gebildet wird, wobei das Molekulargewicht des PVA bzw. der Mischung so gewählt wird, dass er gegebenenfalls nach Aufhebung der Vernetzung, im wesentlichen ohne Degradation der PVA-Moleküle über die Niere ausscheidbar ist.

Mit besonderem Vorteil wird das medizintechnische Produkt lyophilisiert. Durch Lyophilisation kann der erfindungsgemässe PVA in eine schwammartige Struktur überführt werden. Eine solche Struktur verbessert die Handhabungseigenschaften des medizintechnischen Produkts.

Gemäß der Erfindung ist PVA physikalisch vernetzt, insbesondere durch Kristallitbildung. Mit Vorteil kann die physikalische Vernetzung durch Gefrier-/Tauzyklen vorgenommen werden, die insbesondere mehrfach wiederholt werden. Bevorzugt können durch Gefrier-/Tauzyklen Nanopartikel hergestellt werden.

Zur Herstellung von PVA-Partikeln wird eine Wasser/Öl-Emulsion homogenisiert und diese Emulsion eingefroren. Es ist keine Tensidzugabe nötig. Die Grösse der resultierenden Partikel hängt von der Dauer und der eingestellten Drehzahl (Umdrehungen pro Minute) im Homogenizer und natürlich der Konzentration an PVA ab. Die Grösse der PVA-Partikel kann im Bereich Nanometer über Mikrometer bis Millimeter eingestellt werden. Die Zahl der Gefrier-Auftau-Zyklen bestimmt den Kristallinitätsgrad und damit das Quellungsverhalten. Nach den Gefrierzyklen können diese PVA-Nanöpartikel oder Mikropartikel mittels Filtration abgetrennt und getrocknet werden. Solche Partikel können beispielsweise als Spray versprüht werden. Mit Vorteil können sie somit in der Adhäsionsprophylaxe eingesetzt werden.

In einer anderen Ausführungsform der Erfindung kann PVA chemisch vernetzt werden, insbesondere in einem Lösemittelgemisch vernetzt werden. Hierbei kann eine unter physiologischen Bedingungen reversible Vernetzung bevorzugt sein. Bevorzugt kann eine chemische Vernetzung mittels mehrwertiger Carbonsäuren, insbesondere ihren Derivaten vorgenommen werden.

Nach dem erfindungsgemässen Verfahren kann das Auflösungsverhalten, insbesondere die Funktionsdauer von physikalisch vernetzten PVA, mit einem Molekulargewicht im Bereich von 15000 bis 400000 g/mol durch Vermischen mit hochmolekularen Komponenten, insbesondere PVA und/oder Zuckerpolymeren auf den gewünschten Grad eingestellt werden.

Mit Vorteil kann die Vernetzung bis zu einem gewünschten Vernetzungsgrad durchgeführt werden. Erfindungsgemäss kann das Auflösungsverhalten, insbesondere die Funktionsdauer durch den Vernetzungsgrad eingestellt werden.

Erfindungsgemäss bevorzugt sind Veresterungsreaktionen, die säurekatalysiert und damit pH-abhängig ablaufen. Ein wichtiger Verfahrensparameter ist die Konzentration von PVA in der wässrigen Reaktionslösung. Beispielsweise ist für einen PVA mit einem Molekulargewicht von 22000 g/mol eine Konzentration in wässriger Lösung von mindestens 6 Gew.-% erforderlich. Die Kondensationsreaktion läuft umso schneller ab, je höher die Temperatur ist. Die Gelbildungszeit hängt vom Anstieg der Viskosität ab. Die Gelbildungszeit nimmt auf die Hälfte ab, wenn die Temperatur um 10 °C steigt. Andererseits steigt die Gelbildungszeit um mehrere Stunden, wenn der pH erhöht wird, beispielsweise von 9 auf 42 Stunden bei pH-Änderung von 1,2 auf 2,9.

In einer Ausführungsform kann die Vernetzungsreaktion an vorgefertigten Erzeugnissen, insbesondere PVA-Filmen durchgeführt werden. Aus PVA in wässriger Lösung können mittels Spin-Casting homogene Filme hergestellt werden. Werden solche Filme nach dem Trocknen in eine Lösungsmittelgemisch aus 90 % Aceton und 10 % Wasser gebracht, lässt das Wasser die PVA-Membran quellen, ohne sie aufzulösen. In eine derart vorgequollene PVA-Membran kann ein chemisches Vernetzungsagens leicht eindringen und nach der vorgesehenen Reaktion wieder ausgewaschen werden.

Des weiteren betrifft die Erfindung die Bereitstellung des medizintechnischen Produkts wie es oben beschrieben ist zur Verwendung bei der Prophylaxe von Adhäsionen bei chirurgischen Eingriffen in der Humanmedizin und Veterinärmedizin. Das erfindungsgemässe Produkt auf Basis von PVA kann je nach den medizinischen Erfordernissen und der gewünschten Einsatzweise in verschiedenen Formen bereitgestellt bzw. zur Verwendung vorbereitet werden. Beispielsweise in Form einer Membran, einer Lösung, eines Schaums, eines Gels oder eines Sprays. Das Produkt kann ganz oder teilweise eingefärbt sein. Das erfindungsgemässe medizintechnische Produkt kann als Beschichtung auf chirurgischen Materialien wie beispielsweise Implantaten vorgesehen sein.

Mit Vorteil kann das medizintechnische Produkt in Form einer Membran zur physikalischen Separation von Gewebeschichten im Körper bestimmt sein.

In einer weiteren Ausführungsform kann das medizintechnische Produkt in Form einer mehrschichtigen Membran oder Folie zur physikalischen Separation von Gewebeschichten im Körper bestimmt sein, wobei vorzugsweise eine Seite so ausgebildet ist, dass Gewebeadhäsionen vermieden werden und die andere so ausgebildet ist, insbesondere durch Strukturierung der Oberfläche, dass Gewebeadhäsionen begünstigt werden. Auf diese Weise kann beim chirurgischen Eingriff ein Annähen der Folie bzw. Membran zum Fixieren im Körper entfallen.

In einer anderen Ausführungsform kann das medizintechnische Produkt in Form einer Lösung zur physikalischen Separation von Gewebeschichten im Körper bestimmt sein.

In einer weiteren Ausführungsform kann das medizintechnische Produkt in Form eines Schaums zur physikalischen Separation von Gewebeschichten im Körper bestimmt sein.

In noch einer anderen Ausführungsform kann das medizintechnische Produkt in Form eines Gels, insbesondere eines Sprühgels zur physikalischen Separation von Gewebeschichten im Körper bestimmt sein.

In noch einer weiteren Ausführungsform kann das medizintechnische Produkt in Form von Nanopartikeln zur physikalischen Separation von Gewebeschichten bestimmt sein.

In einer anderen Ausführungsform kann das medizintechnische Produkt als Spray zur physikalischen Separation von Gewebeschichten bestimmt sein.

In einer speziellen Ausführungsform kann das medizintechnische Produkt mit einer separaten bioverträglichen Komponente in Zusammenwirkung zur physikalischen Separation von Gewebeschichten bestimmt sein. Als Beispiel kann eine Wechselwirkung von PVA mit ionischen Komponenten genannt werden, wobei sich bei deren Kombination im Operationsgebiet über lonenbrücken ein Gel bildet, das zur Adhäsionsprophylaxe dient.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

Herstellung von Membranen und Hydrogelen aus PVA

### Beispiel 1.

### Giessverfahren

PVA wird in ein verschraubbares Schottglas eingewogen mit deionisiertem Wasser aufgefüllt und im Trockenschrank bei 90 °C gelöst. Auf diese Weise ist der Vorgang sehr schnell und Schaumbildung kann vermieden werden (eine Entgasung ist nicht notwendig). Die Lösungen werden mittels einer Einwegspritze durch sterile Filter (0,45 µm Porendurchmesser) filtriert, auf Teflon gegossen und im Laminarflow getrocknet. Die gewünschte Membrandicke kann mit der eingefüllten Menge gesteuert werden. Am besten eignen sich wässrige PVA-Lösungen von 4-20 %. Die Membranen können auf die geeignete Grösse zurechtgeschnitten werden.

Wenn eine strukturierte Oberfläche gewünscht ist, kann im Teflon eine ausgewählte Struktur vorgeformt sein, die sich dann auf das Produkt überträgt.

### Beispiel 2.

### PVA-Viskosität

Der entscheidende Parameter für die Verarbeitung von gelösten hochmolekularen Stoffen ist die Viskosität. 8-10 %ige PVA-Lösungen lassen sich filtrieren und sind auch gut handhabbar. Die Filtration der Lösungen über die 0,45 µm-Filter ist bis zu einer Viskosität von 25 mPas möglich, für höhere Viskositäten müssen Filter mit grösserem Porendurchmesser verwendet werden. Das Giessen ist bis 50 mPas völlig unproblematisch, für höhere Viskositäten sollte sehr langsam gegossen werden und eventuell auftretende Luftblasen mit einer Nadel angestochen bzw. mit einem über die Oberfläche gezogenen Schieber an den Rand transportiert werden.

### Beispiel 3.

### Membraneigenschaften

Die Membranen sind glatt und transparent, sie können jedoch die Struktur der Unterlage falls gewünscht aufnehmen und deshalb milchig erscheinen. Über eine Konditionierung (d.h. Wasseraufnahme) im Klimaschrank kann dies wieder aufgehoben oder eingestellt werden. Die Membranen sind homogen und zeigen bis zu einer 1500fachen Vergrösserung keine Poren. Im REM sind keine Löcher zu entdecken, um das Bild scharf zu stellen, müssen Staubkörner oder ähnliches fokussiert werden.

### Beispiel 4.

### Membran-Konditionierung

Die PVA-Membranen werden durch lange Trocknungsphasen an der Luft relativ spröde. Eine Konditionierung unter definierten Wärme- und Feuchtigkeitsbedingungen ist unerlässlich. Der Klimaschrank wird auf 37 °C und 100 % Luftfeuchtigkeit eingestellt. Unter der Einwirkung der Feuchtigkeit werden die Membranen sehr schnell weich und flexibel. Um optimale Konditionierungsbedingungen zu finden, wurden von verschiedenen Probenzusammensetzungen Teststreifen (4x1 cm) geschnitten und im Klimaschrank unterschiedlich lange gelagert. Untersuchungen der mechanischen Eigenschaften zeigten, dass innerhalb der ersten drei Tage im Klimaschrank die Reisskraft abnimmt. Nach etwa 7 Tagen sind die Membranen abgesättigt und die Reisskraft pendelt sich auf einen Plateauwert ein. Die Reisskraft liegt dann im Bereich der Hälfte des Ausgangswertes. Eine Fehlerermittlung mit je 20 Proben aus drei Chargen ergibt nach 3 Tagen Konditionierung einen Fehlerbereich von 10 N/mm2 (delta = 10 N/mm2). Die Dehnung der Proben nimmt mit der Wasseraufnahme deutlich zu. Der Fehlerbereich, ebenfalls nach 3 Tagen Klimaschrank, liegt bei 30 % (delta = 30 %). Die Konditionierung erhöht die Dehnung der Proben auf bis zu 500 %. Das heisst, dass die Membranen im relevanten Zeitraum die Bewegungen und damit verbundenen Dehnungen, die durch normale Körper- und Darmbewegungen entstehen, ohne Rissbildung überstehen. Bereits nach einem Tag Lagerung im Klimaschrank erhöht sich die Dehnbarkeit der Filme auf mehr als das Doppelte, ein Plateauwert wird (wie auch bei der Messung der Reisskraft) nach etwa 6 bis 7 Tagen gefunden.

### Beispiel 5.

### Zusatzstoffe

Eine Beimischung von wenigen Prozent höhermolekularem PVA äussert sich in den Dehnungsmessungen deutlich. Schon die Zugabe von 0,5 bis 1 Gew-% höhermolekularem PVA erhöht die Dehnbarkeit der Membranen um bis zu 100 %. Eine Verstärkung kann also durch Mischen verschiedener PVAs mit unterschiedlichen Molekulargewichten erreicht werden.

### Beispiel 6.

### Resorptionsverhalten

Die Resorbierbarkeit der Membranen mit unterschiedlichen Dicken (von 0,06 mm bis 0,16 mm) wurde bei 37 °C in wässriger Lösung untersucht. Die zeitabhängige Auflösung der verschiedenen Membranen kann durch Variation der Zusammensetzung und der Dicke gesteuert werden. Membranen, die nur aus relativ kurzem PVA bestehen (Mw = 20000 g/mol), sind ab einer Dicke von 0,12 mm über einen Zeitraum von 4 Wochen stabil. Dünnere Filme lösen sich schnell auf, sie zerfallen zuerst in grössere Bruchstücke, die aber keinen vollständigen Schutz vor Adhäsionen bieten können. Für homologe Mischungen, also Blends aus PVA mit einer hochmolekularen Komponente sind Membranen mit der Dicke ≥ 0,1mm über einen Zeitraum von 4 Wochen stabil. Sowohl die reinen PVA-Filme als auch die Blends erscheinen in der Lösung transparent.

### Beispiel 7.

### Einfluss von Zusatzstoffen auf die Resorption

Wird Carboxymethylcellulose (kurz CMC) oder andere Kohlenhydrate zugemischt, ändert sich die Resorptionsgeschwindigkeit und der Zerfallsmechanismus. Die Filme quellen mehr auf und erscheinen trübe, sie zerfallen nicht in grössere Bruchstücke. Es bilden sich auf der Oberfläche der Membran kleinere Löcher, die immer grösser werden. Schon die Beifügung von 0,25 Gew-% CMC ändert das Zerfallsverhalten der Membranen.

### Beispiel 8.

### Sterilisation

Als Sterilisationsmethoden wurden Gammabestrahlung, EtOxSterilisation und Plasmasterilisation überprüft. Eine Gefahr bei der Sterilisation besteht darin, dass im Material Radikale gebildet werden können, die dann zu stabilen Kohlenstoff-Kohlenstoff-Verknüpfungen innerhalb der Membran führen können. Eine Folge davon wäre eine Unlöslichkeit des Materials und damit eine völlig veränderte Resorption. Solche neuen Bindungen im Material äussern sich jedoch dramatisch in der Viskosität. Zur Überprüfung wurden deshalb die sterilisierten Membranen wieder gelöst und im Viscotester gemessen. Die Viskositäten der Membranen nach der Gammasterilisation liegen unter den Ausgangswerten, das kann damit erklärt werden, dass die Filme Wasser enthalten und somit die Einwaage etwas verfälschen. Die Co60-Strahlung lag im Bereich von 26,2 bis 30,4 kGy. Die Plasmasterilisation beinhaltet noch Trocknungsschritte im Vakuum, aus diesem Grunde erhält man hier eine sehr gute Übereinstimmung der Viskosität mit der 8 %igen Ausgangslösung. Eine Vernetzung ist in keinem Fall (Überprüfung mehrerer Chargen) zu erkennen. Alle Sterilisationsmuster waren steril, d.h. alle drei Methoden können eingesetzt werden.

### Beispiel 9.

### Bioverträglichkeit

Die Biokompatibilität der untersuchten Membranen ist ausgezeichnet, z.B. zeigt die akute systemische Toxizitätsprüfung in Mäusen keinerlei Unterschied zu den injizierten KontrollLösungen. Die Injektionen wurden teilweise intravenös und teilweise intraperitoneal durchgeführt. Die Tiere wurden nach 4, 24, 48 und 72 Stunden nach der Injektion beobachtet. Dasselbe gilt z.B. auch für die Cytotoxizitätsprüfungen mit Mausfibroblasten, welche ebenfalls gute Ergebnisse zeigten. Es wurde keinerlei Veränderung der Zellmorphologie oder Hinweise toxischer Wirkung gefunden. Das Material ist nicht zytotoxisch und weist keine akute systemische Toxizität auf.

### Beispiel 10.

### Hydrogele

PVA-Hydrogele können über Einfrier-Auftau-Prozesse hergestellt werden. 20 %ige PVA Lösungen werden in einer Petrischale bei 20 Grad eingefroren. Dieser Vorgang kann in mehreren Zyklen durchgeführt werden: 12 Stunden einfrieren und in abgedecktem Zustand wieder 2 Stunden auftauen. Die resultierenden PVA-Hydrogele sind formstabil und adhäsiv. Je höher das Molekulargewicht des PVA, desto formstabiler ist das resultierende Gel.

### Beispiel 11.

### Nanopartikel

Nach den in Beispiel 10 genannten Gefrier-/Tauzyklen können auch PVA-Partikel hergestellt werden. PVA oder PVA-Mischungen (verschiedene Molekulargewichte) werden in Wasser oder Phosphat-Puffer von pH 7,4 gelöst. Die wässrige Lösung wird in einem Volumenanteil von 2 bis 20 % in einen grossen Überschuss von Silikonöl gebracht. Dieses Gemisch wird in einem Homogenizer 5 Minuten lang bei 10000 Umdrehungen pro Minute zu einer Wasser/Öl-Emulsion homogenisiert. Nach 1 bis 10 Gefrierzyklen bei -20 °C werden die PVA-Partikel mit Aceton im Volumenverhältnis 1:10 extrahiert. Die PVA-Partikel werden abfiltriert, mit Aceton gewaschen und im Vakuum getrocknet. Die Form der Nanopartikel ist fast rund, mit etwas rauher Oberfläche. PVA-Hydrogel-Nanopartikel sind im Durchschnitt 680 ± 40 nm gross.

### Beispiel 12.

### Verbesserung der Gewebeadhäsion

Es wurden Untersuchungen zur Gewebeadhäsion von im Laminarflow getrockneten PVA-Membranen oder von durch Gefrier-/Tauzyklen hergestellten formstabilen PVA-Hydrogelen vorgenommen.

Das Adhäsionsverhalten der PVA-Produkte kann durch Aufrauhung der Oberfläche verbessert werden. Dies kann durch Giessen des PVA auf Unterlagen mit definierten Oberflächen erreicht werden. Es kann hierzu eine geriffelte Unterlage verwendet werden. Ebenso können in die PVA-Oberfläche nachträglich Störungen aufgebracht werden, beispielsweise mit Nadeln im Mikrometerbereich oder durch Einpressen eines Musters.

Die Gewebeadhäsion kann auch durch eine Vernetzungsgradientenbildung verbessert werden. Dies kann durch eine Einstellung verschiedener Temperaturen schon während der Gelbildung erreicht werden. Auch durch chemische Modifikation des PVA kann die Adhäsion verbessert werden. Die Hydrophilie der Membranoberfläche kann durch Einbringen von funktionellen Gruppen wie Carboxylgruppen verstärkt werden. Ferner kann die Hydrophilie der PVA-Membran durch die Art der Unterlage, auf die sie gegossen wurde, beeinflusst werden. So erreicht ein Giessen auf polare Oberflächen wie Glas eine verstärkte Hydrophilie.

Bei durch Gefrier-Auftau-Zyklen hergestellten PVA-Hydrogelen kann durch Gefriertrocknen im Lyophilisator eine Schwammstruktur hergestellt werden. Eine solche schwammartige Struktur verbessert das Handling und die Adhäsion auf feuchtem Gewebe, ohne die Wirksamkeit zu beeinträchtigen. Durch weitere Verarbeitungsprozesse, wie Pressen, kann das Verhalten der schwammartigen Struktur dem Gewebe angepasst werden.

Durch Lösungsmittelverdampfen hergestellte PVA-Membranen können mit PVA-Lösung besprüht und anschliessend lyophilisiert werden. Die gebildete Doppelschicht bringt den Vorteil einer kompakten Membran einerseits und einer bioadhäsiven schwammartigen Struktur andererseits.

Über Gefrier-/Tauzyklen hergestellte PVA-Hydrogele können einem Temperaturgradienten ausgesetzt werden, so dass bioadhäsive Eigenschaften im Material ausgebildet werden. Die vorteilhaften Eigenschaften ergeben sich durch unterschiedliche Dichte der physikalischen Netzpunkte aufgrund des Gradienten.

PVA-Lösungen können über Düsen versprüht und so zu einem Vlies verarbeitet werden, das mit Vorteil in der Adhäsionsprophylaxe zu verwenden ist.

### Beispiel 13.

### PVA-Beschichtung

In der Chirurgie werden textile Netze aus Polypropylen als Implantate verwendet, die sehr oft in direkten Kontakt mit dem viszeralen Abdomen kommen, wobei es bei Traumatisierung des Körpergewebes zu Verwachsungen kommen kann. Eine Beschichtung des vorgefertigten PP-Netzes mit einer anti-adhäsiven PVA-Schicht gemäss der Erfindung kann solchen Verwachsungen entgegenwirken. Die Schicht sollte bis zur Abheilung des Gewebes, etwa 7 Tage, am Ort verbleiben und danach resorbiert werden.

Die Beschichtung der Netze erfolgt durch Eintauchen in eine PVA-Lösung. Die Schichtdicke auf dem PP-Netz kann durch die Konzentration der PVA-Lösung oder die Eintauchzeit gesteuert werden.

Eine sehr dünne Schicht im Nanometerbereich erreicht man durch Eintauchen des Netzes in eine 1 %ige etwa 80 °C heisse PVA-Lösung. Für den Beschichtungsvorgang spielt das Molekulargewicht des PVAs keine entscheidende Rolle. Jedoch hängt die Resorptionszeit im Körper direkt mit dem Molekulargewicht zusammen. Für sehr dünne Beschichtungen ist es deshalb bevorzugt, hochmolekularen PVA von etwa 200000 g/mol Molekulargewicht einzusetzen, damit sich die Schicht nicht sofort wieder löst und damit keinen Schutz vor Adhäsionen bieten kann. Eine sehr dünne Beschichtung verändert die Materialeigenschaften des Netzes nicht und die PVA-Schicht ist transparent.

Für eine dickere Schicht im Mikrometerbereich wird das zu beschichtende Netz einmal oder mehrmals in eine 3 bis 10 %ige PVA-Lösung eingetaucht und eingefroren. In ein bis fünf Frost-Tau-Zyklen, je nach gewünschter Festigkeit, bildet sich so ein PVA-Hydrogel. Das PVA-Hydrogel ist elastisch und verändert auch in dickerer Schicht die Materialeigenschaften des Netzes nicht. Die Resorptionszeit wird durch diese physikalische Vernetzung entscheidend verlängert. Je höher das Molekulargewicht, umso stabiler ist das Hydrogel. Zur genauen Einstellung der Resorptionszeit kann auch eine Mischung aus niedermolekularem/hochmolekularem PVA eingesetzt werden. Die PVA-Schicht ist transparent.

### Ergebnis der Prophylaxe

Einsatz bei chirurgischem Eingriff im Tierversuch

### Beispiel 14.

Als Tiermodell wird das Zökum von Hasen verwendet.

Die Oberfläche des Zökums (Caecum) wird mit einer Gaze-Kompresse 15 min abgerieben. Das viszerale Peritoneum, das dem übrigen Darmkonvolut zugewandt ist, wurde über 5 Minuten abgerieben. Geprüft wurden der prozentuale Anteil der Verwachsungsfläche zwischen der Wundfläche im parietalen Peritoneum und dem viszeralen Peritoneum auf der Zökumoberfläche, die Tenazität der Verwachsung sowie der histologische Zustand im Bereich des Bauchwanddefekts.

Als Adhäsionsprophylaxe werden verwendet:
ein PVA-Film aus 8 % Lösung im Laminarflow getrocknet, in einer Dicke von 0,09 bis 0,1 mm (AAF1),
ein PVA-Hydrogel aus PVA1/PVA2 im Gewichtsverhältnis 60:40 in einer Dicke von 0,15 mm hergestellt durch Gefrier-AuftauZyklen,
sowie ein handelsüblicher Antiadhäsionsfilm als Referenz.

Das erfindungsgemässe Hydrogel umfasst mit PVA1 relativ kurzkettigen PVA mit einem Molekulargewicht von ca. 20000 g/mol und mit PVA2 relativ langkettigen PVA mit einem Molekulargewicht von ca. 200000 g/mol. Das Hydrogel wurde hergestellt aus 20 % wässriger Lösung unter 3maliger Durchführung der Gefrier-/Tauprozesse. Das formstabile Hydrogel besteht zu etwa 80 % aus Wasser.

Die prophylaktisch unbehandelte Kontrollgruppe von 12 Tieren zeigte Adhäsion zu 100 % Flächenausmass. Die Proben AAF1 und AAF2 zeigen eine bessere Wirksamkeit als der bekannte Antiadhäsionsfilm.

Bei Prophylaxe mit AAF1 sind von 12 Tieren 8 Tiere vollkommen adhäsionsfrei, 4 Tiere zeigen Adhäsionen mit Flächenausmass von 50, 15, 2 bzw. 10 %. Bei Prophylaxe mit AAF2 zeigen von 12 Tieren 6 Tiere Adhäsionen mit geringem Flächenausmass von 3 bis 10 %. Eine statistische Analyse der Daten im One Way Analysis of Variance Test (ANOVA) zeigt einen signifikanten Unterschied zur Kontrollgruppe. Im Vergleich zum bekannten Antiadhäsionsfilm reduzieren AAF1 und AAF2 die Adhäsionflächen. AAF1 und AAF2 unterscheiden sich nicht signifikant voneinander, jedoch sind die Unterschiede zum bekannten Film signifikant.

Bei den Tiergruppen, die mit AAF1 und AAF2 behandelt wurden, sind die Verwachsungen, sofern welche auftreten, zarte und leicht lösbare Bindegewebsstrukturen. Die PVA-Folien sind also wirksam zur Adhäsionsprophylaxe.

### Beispiel 15.

Als Tiermodell wird das Uterus-Horn-Modell an Hasen gebraucht, das ein in der Literatur etabliertes Adhäsionssystem mit langer Erfahrung ist.

Die Serosa am Uterushorn wurde auf einer Länge von 5 cm mit einem frischen Skalpell abgeschabt. So sind punktuelle Blutungen generiert worden. Insgesamt wurden 12 Hasen operiert. 8 Tiere wurden mit AAF2 prophylaktisch behandelt und 4 Tiere dienten als Kontrollgruppe. Alle Tiere hielten ihr Gewicht oder nahmen geringfügig zu und zeigten keine Anzeichen akuter Toxizität. Von den 8 mit Adhäsionsprophylaxe behandelten Tieren zeigten 7 keine Adhäsion, eines zeigte leichte Verwachsungen auf etwa 25 % der Fläche. Durch die PVA-Adhäsionsprophylaxe wird deutliche Reduktion der Adhäsionen erreicht.

## Patentansprüche

1. Medizintechnisches Produkt zur Adhäsionsprophylaxe für die postoperative Verhinderung von Verwachsungen im Körper umfassend einen PVA (Polyvinylalkohol) mit einem Molekulargewicht von 15 000 bis 400 000 g/mol, der physikalisch vernetzt ist.

2. Produkt nach Anspruch 1, **dadurch gekennzeichnet, dass** die physikalische Vernetzung durch Kristallitbildung vorgenommen ist.

3. Produkt nach Anspruch 1, **dadurch gekennzeichnet, dass** der PVA ein Molekulargewicht von 20 000 bis 400 000 g/mol aufweist.

4. Produkt nach einem der Ansprüche 1 bis 3, **dadurch gekennzeichnet, dass** der PVA aus einem Gemisch von niedermolekularen und hochmolekularen Komponenten gebildet ist, von denen mindestens eine PVA ist, wobei die hochmolekulare Komponente insbesondere hochmolekularer PVA ist.

5. Produkt nach einem der vorhergehenden Ansprüche, **dadurch gekennzeichnet, dass** PVA in Mischung mit einer hochmolekularen Komponente, die kein PVA ist, vorliegt.

6. Produkt nach Anspruch 5, **dadurch gekennzeichnet, dass** die hochmolekulare Komponente in einer Menge von 0,5 bis 4 Gew.-%, insbesondere 1 bis 2 Gew.-% vorliegt.

7. Produkt nach Anspruch 5 oder 6, **dadurch gekennzeichnet, dass** dem PVA als hochmolekulare Komponente ein Zuckerpolymer zugesetzt ist.

8. Produkt nach Anspruch 7, **dadurch gekennzeichnet, dass** das Zuckerpolymer ausgewählt ist aus der Gruppe bestehend aus Carboxymethylcellulose, Dextran und/oder Hydroxymethylcellulose.

9. Produkt nach einem der vorhergehenden Ansprüche, **dadurch gekennzeichnet, dass** es in Form von Mikropartikeln, insbesondere Nanopartikeln vorliegt.

10. Produkt nach einem der vorhergehenden Ansprüche, **dadurch gekennzeichnet, dass** es in mit wässrigen Medien gequollener Form bereitgestellt ist.

11. Produkt nach einem der vorhergehenden Ansprüche, **dadurch gekennzeichnet, dass** das Molekulargewicht des PVA bzw. der Mischung so gewählt ist, dass er gegebenenfalls nach einer Hydrolyse bzw. Aufhebung der Vernetzung, im wesentlichen ohne Degradation der PVA-Moleküle über die Niere ausscheidbar ist.

12. Produkt nach einem der vorhergehenden Ansprüche, **dadurch gekennzeichnet, dass** seine Funktionsdauer im Operationsgebiet 5 bis 21 Tage, insbesondere 5 bis 14 Tage beträgt.

13. Produkt nach einem der vorhergehenden Ansprüche, **dadurch gekennzeichnet, dass** seine makroskopische Auflösung unter physiologischen Bedinungen 7 bis 60 Tage beträgt.

14. Produkt nach einem der Ansprüche 1 bis 13 zur Verwendung bei der Prophylaxe von Adhäsionen bei chirurgischen Eingriffen in der Humanmedizin und Veterinärmedizin.

15. Produkt nach Anspruch 14, **dadurch gekennzeichnet, dass** das medizintechnische Produkt in Form von Nanopartikeln zur physikalischen Separation von Gewebeschichten bestimmt ist.

16. Produkt nach Anspruch 14 oder 15, **dadurch gekennzeichnet, dass** das medizintechnische Produkt als Spray zur physikalischen Separation von Gewebeschichten bestimmt ist.

17. Verfahren zur Herstellung eines medizintechnischen Produkts zur Adhäsionsprophylaxe, **dadurch gekennzeichnet, dass** PVA (Polyvinylalkohol) mit einem Molekulargewicht von 15 000 bis 400 000 g/mol physikalisch vernetzt wird, wobei das Molekulargewicht des PVA bzw. der Mischung so gewählt ist, dass er gegebenenfalls nach Aufhebung der Vernetzung, im wesentlichen ohne Degradation der PVA-Moleküle über die Niere ausscheidbar ist.

18. Verfahren nach Anspruch 17, **dadurch gekennzeichnet, dass** PVA durch Kristallitbildung physikalisch vernetzt wird.

19. Verfahren nach einem der Ansprüche 17 oder 18, **dadurch gekennzeichnet, dass** die physikalische Vernetzung durch Gefrier-/Tauzyklen vorgenommen wird, die insbesondere mehrfach wiederholt werden.

20. Verfahren nach einem der Ansprüche 17 bis 19, **dadurch gekennzeichnet, dass** durch Gefrier-/Tauzyklen Nanopartikel hergestellt werden.

21. Verfahren nach einem der Ansprüche 17 bis 20, **dadurch gekennzeichnet, dass** das medizintechnische Produkt lyophilisiert wird.

22. Verfahren nach einem der Ansprüche 17 bis 21, **dadurch gekennzeichnet, dass** das Auflösungsverhalten insbesondere die Funktionsdauer von physikalisch vernetztem PVA durch Vermischen mit hochmolekularen Komponenten, insbesondere PVA und/oder Zuckerpolymeren auf den gewünschten Grad eingestellt wird.

23. Verfahren nach einem der Ansprüche 17 bis 22, **dadurch gekennzeichnet, dass** das Auflösungsverhalten, insbesondere die Funktionsdauer durch den Vernetzungsgrad eingestellt wird.

## Claims

1. Medical technology product for the prophylaxis of adhesions for the post-operative prevention of adhesions in the body, comprising a PVA (polyvinyl alcohol) which has a molecular weight of from 15 000 to 400 000 g/mol and which is physically crosslinked.

2. Product according to Claim 1, **characterized in that** the physical crosslinking is performed by means of crystallite formation.

3. Product according to Claim 1, **characterized in that** the PVA has a molecular weight of from 20 000 to 400 000 g/mol.

4. Product according to any of Claims 1 to 3, **characterized in that** the PVA is formed from a mixture of low-molecular-weight and high-molecular-weight components, at least one of which is PVA, wherein the high-molecular-weight component is more particularly high-molecular-weight PVA.

5. Product according to any of the preceding claims, **characterized in that** PVA is present in a mixture having a high-molecular-weight component which is not PVA.

6. Product according to Claim 5, **characterized in that** the high-molecular-weight component is present in an amount of from 0.5% to 4% by weight, more particularly from 1% to 2% by weight.

7. Product according to Claim 5 or 6, **characterized in that** a sugar polymer is added to the PVA as a high-molecular-weight component.

8. Product according to Claim 7, **characterized in that** the sugar polymer is selected from the group consisting of carboxymethylcellulose, dextran and/or hydroxymethylcellulose.

9. Product according to any of the preceding claims, **characterized in that** it is present in the form of microparticles, more particularly nanoparticles.

10. Product according to any of the preceding claims, **characterized in that** it is provided in a form swollen with aqueous media.

11. Product according to any of the preceding claims, **characterized in that** the molecular weight of the PVA or of the mixture is selected such that the PVA, after any hydrolysis or reversal of crosslinking, is excretable via the kidneys essentially without degradation of the PVA molecules.

12. Product according to any of the preceding claims, **characterized in that** its functional life in the area of the surgical intervention is from 5 to 21 days, more particularly from 5 to 14 days.

13. Product according to any of the preceding claims, **characterized in that** its macroscopic dissolution under physiological conditions takes from 7 to 60 days.

14. Product according to any of Claims 1 to 13 for use in the prophylaxis of adhesions in surgical procedures in human and veterinary medicine.

15. Product according to Claim 14, **characterized in that** the medical technology product in the form of nanoparticles is intended for the physical separation of tissue layers.

16. Product according to Claim 14 or 15, **characterized in that** the medical technology product in the form of a spray is intended for the physical separation of tissue layers.

17. Method for producing a medical technology product for the prophylaxis of adhesions, **characterized in that** PVA (polyvinyl alcohol) having a molecular weight of from 15 000 to 400 000 g/mol is physically crosslinked, wherein the molecular weight of the PVA or of the mixture is selected such that the PVA, after any reversal of crosslinking, is excretable via the kidneys essentially without degradation of the PVA molecules.

18. Method according to Claim 17, **characterized in that** PVA is physically crosslinked by means of crystallite formation.

19. Method according to either of Claims 17 and 18, **characterized in that** the physical crosslinking is performed by means of freeze/thaw cycles which in particular are repeated several times.

20. Method according to any of Claims 17 to 19, **characterized in that** nanoparticles are produced by means of freeze/thaw cycles.

21. Method according to any of Claims 17 to 20, **characterized in that** the medical technology product is lyophilized.

22. Method according to any of Claims 17 to 21, **characterized in that** the dissolution behaviour, more particularly the functional life of physically crosslinked PVA, is set to the desired level by mixing with high-molecular-weight components, more particularly PVA and/or sugar polymers.

23. Method according to any of Claims 17 to 22, **characterized in that** the dissolution behaviour, more particularly the functional life, is set by means of the degree of crosslinking.

## Revendications

1. Produit médico-technique destiné à la prophylaxie d'adhérences pour la prévention postopératoire d'adhérences dans le corps, comprenant un APV (alcool polyvinylique) avec un poids moléculaire de 15 000 à 400 000 g/mole, qui est physiquement réticulé.

2. Produit selon la revendication 1, **caractérisé en ce que** la réticulation physique est effectuée par formation de cristallites.

3. Produit selon la revendication 1, **caractérisé en ce que** le APV présente un poids moléculaire de 20 000 à 400 000 g/mole.

4. Produit selon l'une quelconque des revendications 1 à 3, **caractérisé en ce que** le APV est formé d'un mélange de composants à bas poids moléculaire et à haut poids moléculaire, dont au moins un est un APV, dans lequel le composant à haut poids moléculaire est en particulier un APV à haut poids moléculaire.

5. Produit selon l'une quelconque des revendications précédentes, **caractérisé en ce que** le APV se présente en mélange avec un composant à haut poids moléculaire, qui n'est pas un APV.

6. Produit selon la revendication 5, **caractérisé en ce que** le composant à haut poids moléculaire est présent en une quantité de 0,5 à 4 % en poids, en particulier de 1 à 2 % en poids.

7. Produit selon la revendication 5 ou 6, **caractérisé en ce qu'**un polymère sucre est ajouté au APV en guise de composant à haut poids moléculaire.

8. Produit selon la revendication 7, **caractérisé en ce que** le polymère sucre est sélectionné dans le groupe composé de la carboxyméthylcellulose, du dextrane et/ou de l'hydroxyméthylcellulose.

9. Produit selon l'une quelconque des revendications précédentes, **caractérisé en ce qu'**il se présente sous la forme de microparticules, en particulier de nanoparticules.

10. Produit selon l'une quelconque des revendications précédentes, **caractérisé en ce qu'**il est préparé sous une forme gonflée avec des milieux aqueux.

11. Produit selon l'une quelconque des revendications précédentes, **caractérisé en ce que** le poids moléculaire du APV ou du mélange est choisi de telle manière qu'il puisse s'éliminer par les reins, éventuellement après une hydrolyse ou une suppression de la réticulation, essentiellement sans dégradation des molécules de APV.

12. Produit selon l'une quelconque des revendications précédentes, **caractérisé en ce que** sa durée fonctionnelle dans la zone opératoire est de 5 à 21 jours, en particulier de 5 à 14 jours.

13. Produit selon l'une quelconque des revendications précédentes, **caractérisé en ce que** sa décomposition macroscopique dans les conditions physiologiques dure 7 à 60 jours.

14. Produit selon l'une quelconque des revendications 1 à 13 à utiliser lors de la prophylaxie d'adhérences lors d'interventions chirurgicales en médecine humaine et en médecine vétérinaire.

15. Produit selon la revendication 14, **caractérisé en ce que** le produit médico-technique sous forme de nanoparticules est destiné à la séparation physique de couches de tissus.

16. Produit selon la revendication 14 ou 15, **caractérisé en ce que** le produit médico-technique sous forme de spray est destiné à la séparation physique de couches de tissus.

17. Procédé de fabrication d'un produit médico-technique destiné à la prophylaxie d'adhérences, **caractérisé en ce que** l'on effectue la réticulation physique de APV (alcool polyvinylique) avec un poids moléculaire de 15 000 à 400 000 g/mole, dans lequel le poids moléculaire du APV ou du mélange est choisi de telle manière qu'il puisse s'éliminer par les reins, éventuellement après suppression de la réticulation, essentiellement sans dégradation des molécules de APV.

18. Procédé selon la revendication 17, **caractérisé en ce que** l'on effectue la réticulation physique du APV par formation de cristallites.

19. Procédé selon l'une des revendications 17 ou 18, **caractérisé en ce que** l'on effectue la réticulation physique par des cycles de congélation-décongélation, qui sont notamment répétés plusieurs fois.

20. Procédé selon l'une quelconque des revendications 17 à 19, **caractérisé en ce que** l'on fabrique des nanoparticules par des cycles de congélation-décongélation.

21. Procédé selon l'une quelconque des revendications 17 à 20, **caractérisé en ce que** l'on effectue la lyophilisation du produit médico-technique.

22. Procédé selon l'une quelconque des revendications 17 à 21, **caractérisé en ce que** l'on règle au degré désiré le comportement de décomposition, en particulier la durée fonctionnelle du APV physiquement réticulé par mélange avec des composés à haut poids moléculaire, en particulier un APV et/ou des polymères sucres.

23. Procédé selon l'une quelconque des revendications 17 à 22, **caractérisé en ce que** l'on règle le comportement de décomposition, en particulier la durée fonctionnelle, au moyen du degré de réticulation.
